# EUROPÄISCHE PATENTSCHRIFT

(11) **EP 1 196 087 B1**
(45) Veröffentlichungstag und Bekanntmachung des Hinweises auf die Patenterteilung: **16.04.2008**
(21) Anmeldenummer: 00943938.1
(22) Anmeldetag: 29.06.2000
(51) Int. Cl.: A61B 5/22

(54) **VERFAHREN ZUR EINSTELLUNG BZW. STEUERUNG VON ERNÄHRUNG UND/ODER VERBRAUCH EINES MENSCHEN**
METHOD FOR ADJUSTING OR CONTROLLING THE DIET AND/OR A PERSON'S CONSUMPTION
PROCEDE POUR LE REGLAGE OU LA COMMANDE DE L'ALIMENTATION OU DE LA CONSOMMATION D'UN ETRE HUMAIN

(30) Priorität: 29.06.1999 DE 19929508; 14.10.1999 DE 19949479
(43) Veröffentlichungstag der Anmeldung: 17.04.2002
(73) Patentinhaber: Stegmann, Heiner, 63450 Hanau (DE)
(72) Erfinder: Stegmann, Heiner, 63450 Hanau (DE)
(74) Vertreter: Stoffregen, Hans-Herbert
(86) Internationale Anmeldenummer: PCT/EP2000/006060
(87) Internationale Veröffentlichungsnummer: WO 2001/000091

(56) Entgegenhaltungen:
- US-A- 4 951 197
- T.L. TALBOT ET AL.: "Noninvasive detection of the anaerobic threshold during computer-controlled exercise testing" MEDICAL & BIOLOGICAL ENGINEERING & COMPUTING, Bd. 23, Nr. 6, November 1985 (1985-11), Seiten 579-584, XP002150591 Stevenage (GB)

## Beschreibung

Die Erfindung bezieht sich auf ein Verfahren zur Einstellung bzw. Steuerung von Ernährung mit Kohlenhydrat- und/oder Fett- und/oder Eiweißanteil eines einer Belastung ausgesetzten Menschen und/oder Verbrauch von Kohlenhydrat- und/oder Fett- und/oder Eiweißanteil eines einer Belastung ausgesetzten Menschen.

Ein solches Verfahren ist aus US 4 95 11 97 bekannt.

Insbesondere bezieht sich die Erfindung auf ein Verfahren zur Ermittlung bedarfsgereckter Diätetika bzw. Nahrungstherapeutika zur Emahrungssteuerung eines Menschen durch die indirekte Bestimmung seines individuellen Kohlenhydrat-, Fett bzw Eiweißanteils an der Energiebereitstellung mittels standardisierter Belastungstests bzw. dem gezielten Verbrauch der diesbezüglichen Anteile unter Berücksichtigung der bei den standardisierten Belastungstests ermittelten Ergebnisse.

Kohlenhydrate, Fette und Eiweiße sind Substrate, die im Muskel zur Energiegewinnimg, d h. ATP-Produktion verstoffwechselt werden. Im Muskel treten beim Übergang vom Ruhezustand zum Zustand starker Belastung große Veränderungen im Stoffwechsel auf. Vor allem steigt aufgrund des gesteigerten Energiebedarfs die Rate des Substratumsatzes stark an.

Von großer Bedeutung ist hierbei, dass sich im Muskel unter Belastung auch die Verhältnisse der Umsatzraten der einzelnen Substrate zueinander stark verändern, d. h. die prozentualen Anteile des Kohlenhydrat-, Fett- bzw. Eiweißumsatzes am Gesamtsubstratumsatz sind im Muskel belastungsspezifisch geregelt.

Der Erfindung liegt das Problem zu Grunde, ein Verfahren der eingangs genannten Art so weiterzubilden, dass mit einfachen Maßnahmen eine zuverlässige Einstellung bzw. Steuerung von Ernährung und/oder Verbrauch eines Menschen in Abhängigkeit von seiner zu berücksichtigenden Belastung in Bezug auf den Kohlenhydrat- und/oder Fett- und/oder Eiweißanteil erfolgt, wobei - insbesondere bei starken Belegen ausgesetzten Personen wie Sportlern oder kranken oder alten Menschen - eine gezielte Einstellung des anzubietenden Kohlenhydrat- und/oder Fett- und/oder Eiweißanteils bzw. dessen Verbrauch erfolgt. Erfindungsgemäß werden zur Lösung des Problems die Verfahrensschritte gemäß Anspruch 1 vorgeschlagen.

Insbesondere ist vorgesehen, dass zur Bestimmung der Leistungsfähigkeit des Menschen Herzfrequenz und/oder Blutdruck und/oder ergospirometrische Parameter und/oder Laktatkonzentration im Blut in Abhängigkeit von der Belastung gemessen bzw. bestimmt werden.

Bevorzugterweise ist erfindungsgemäß vorgesehen, dass zur Bestimmung der Leistungsfähigkeit oberhalb der individuellen anaeroben Schwelle eine Normierung auf eine Laktat-Akkumulationsrate ΔA erfolgt, wobei insbesondere die Laktat-Akkumulationsrate ΔA zur Bestimmung der Ernährung und/oder des Verbrauchs des Menschen in Bezug auf seinen Eiweißanteil aus glucogenen Aminosäuren zu Grunde gelegt wird.

Dabei zeichnet sich ein Verfahren zur Bestimmung der Laktat-Akkumulationsrate ΔA durch die folgenden Verfahrensschritte aus:
- Messung der zeitabhängigen Laktat-Konzentrationsänderung über die individuelle anaerobe Schwelle hinaus,
- Anpassen einer Messkurve an so gewonnene Messwerte, in der die Laktat-Konzentration gegenüber der Zeit aufgetragen wird,
- Bestimmung einer ersten Steigung der Messkurve in einem der individuellen anaeroben Schwelle entsprechenden Zeitpunkt t_{IAT},
- Bestimmung zumindest einer weiteren Steigung aus der Messkurve zu einem Zeitpunkt tₓ mit tₓ > t_{IAT} und
- Subtraktion der zweiten Steigung von der ersten Steigung zur Bestimmung einer Differenz, die die Laktat-Akkumulationsrate ΔA ist.

Um über die belastungsspezifische Regelung des Substratmetabolismus von Probanden Aussagen machen zu können, bedarf es zunächst der Bestimmung der Leistungsfähigkeit bzw. Belastbarkeit dieser Probanden mittels eines standardisierten Tests, der die Möglichkeit der Abschätzung des aeroben/anaeroben Übergangs beinhaltet. Solche Tests sind mit ganz unterschiedlichen Methoden durchführbar.

Es können zur Bestimmung der Leistungsfähigkeit Belastungsarten angewendet werden wie Lauftests, Schwimmtests, Steptests, Ergometrieverfahren, z. B. Fahrrad-, Laufband-, Ruder-Ergometrie mit stufenweiser bzw. kontinuierlicher Belastungssteigerung, Durchführung mit bzw. ohne Pausen.

Alternativ können zur Bestimmung der Leistungsfähigkeit folgende gemessene bzw. aus den Messparametern abgeleitete Parameter verwendet werden:
- *Herzfrequenz (HF) unter Belastung*
   HF-max (bei Ausbelastung)
   HF-submax (anaerob-aerober Übergang: Conconi-Test)
   HF-bezogene Leistung (Physical-Working-Capacity)
   HF-bezogene Sauerstoffaufnahme
- *Blutdruck (RR)*
   Belastungsblutdruck (systolisch)
   Blutdruckamplitude
- *Ergospirometrische Parameter*
   Atemminutenvolumen (AMV)
   Sauerstoffaufnahme (VO₂)
   maximale VO₂ (VO₂-max)
   Atemfrequenz (AF)
   Kohlendioxidabgabe (VCO₂)
   Atemäquivalent (AÄ = AMV / VO₂)
   Sauerstoffpuls (VO₂ / HF)
   Säure-Basen-Status, pH-Wert
   Respiratorischer Quotient (RQ)
   Ventilatorisches Äquivalent für CO₂ und O₂
   Anaerobic Treshold (nach Wasserman)
   Individual Anaerobic Treshold (nach Stegmann)
- *Laktat-Konzentration im Blut*
   IATs nach Stegmann
   ΔA nach Stegmann
   Laktat-Schwellenkonzepte mit fixen Laktatkonzentration und Steigungen
   aus Laktat-Kurve abgeleitete modellbezogene Parameter
   aus Laktat-Kurve und ergospirometrischen Daten abgeleitete Parameter.

Die genaueste Methode stellt allerdings die Ermittlung der Laktat-Leistungskurve im Stufentest mit Bestimmung der individuellen anaeroben Schwelle nach Stegmann (IATs) sowie der IATs-bereinigten Laktat-Akkumulationsrate ΔA dar.

Die Laktat-Leistungskurve eines Menschen ist nur sehr langsam durch Training bzw. Lebensweise veränderbar. Aus ihrem Verlauf können daher Informationen über das Leistungs-und Trainingsverhalten eines Menschen über einen längeren Zeitraum abgeleitet werden, d. h., die Laktat-Leistungskurve eines Menschen kann als "mittelfristiges Gedächtnis" seiner Lebensweise interpretiert werden.

Bezogen auf die IATs bzw. den ΔA-Wert eines Probanden und unter Berücksichtigung obiger Erläuterungen lassen sich folgende allgemeine Aussagen bezüglich des Kohlenhydrat-, Fett- bzw. Eiweißverbrauchs eines Probanden unter Belastung machen (ΔA_{MAX} = größter bei einem Probanden bestimmbarer ΔA-Wert)

| ***Belastungsintensität*** | ***Dauer*** | ***KH*** | ***Fett*** | ***Eiweiß*** |
|---|---|---|---|---|
| Start und Stufenbeginn | sehr kurz [s] | + | ++ | +++ |
| > IATs, ΔA → ΔA_{MAX} | kurz [≤ min] | + | ++ | +++ |
| > IATs, ΔA << ΔA_{MAX} | kurz [6-10min] | ++ | ++ | ++ |
| ≤ IATs | kurz [> 2 min] | ++ | ++ | + |
| ≤ IATs | mittel [< 60 min] | ++ | ++ | + |
| ≤ IATs | lang [> 60 min] | ++ | +++ | ++ |

Die relativen Belastungsintensitäten und die Belastungsdauern, denen ein Mensch z. B. im Alltag oder während sportlicher Betätigung ausgesetzt ist, regulieren folglich die Verhältnisse der Kohlenhydrat-, Fett, und Eiweißanteile an seinem Nährstoffverbrauch. Die diesbezüglichen Verhältnisse werden in der zuvor wiedergegebenen Tabelle grob aufgezeigt. Diese Ergebnisse lassen sich unmittelbar bei der Entwicklung bedarfsgerechter, auf die individuelle Leistungsfähigkeit abgestimmter Formula-Diäten bzw. Nahrungstherapeutika anwenden, um gezielt Ernährungsmängel zu verhindern.

Auch ergibt der Bedarf an Kohlenhydrat-, Fett- und Eiweißanteilen in Anhängigkeit von Belastungsintensität und Belastungsdauer - entsprechend der Tabelle - abgestellt auf die individuelle anaerobe Schwelle bzw. die bereinigte Laktatakkumulationsrate ΔA die Möglichkeit, einen Probanten gezielt derart zu belasten, dass im gewünschten Umfang Kohlenhydrat- und/oder Fett-Anteile abgebaut werden.

Die erfindungsgemäße Lehre stellt folglich eine Verbindung zwischen den Kenntnissen über den belastungsspezifischen Substratverbrauch, d. h. Kohlenhydrat-, Fett- bzw. Eiweißverbrauch eines Menschen und der Möglichkeit dar, diese Spezifität anhand von Leistungstests zu bewerten und daraus individuelle Emährungsempfehlungen abzuleiten bzw. durch geeignete Wahl von Trainingsmodi den Substratverbrauch gezielt zu steuern.

## Patentansprüche

1. Verfahren zur Einstellung des Substratmetabolismus eines Menschen unter Berücksichtigung einer oder mehrerer charakteristischer Parameter wie Blutdruck,
**dadurch gekennzeichnet,**
**dass** die Leistungsfähigkeit des einer Belastung ausgesetzten Menschen durch Bestimmung von für die Leistungsfähigkeit charakteristischen Parametern nicht invasiv ermittelt wird und in Abhängigkeit von der ermittelten Leistungsfähigkeit der Kohlenhydrat- und Fett- und Eiweißanteil-Bedarf individuell bestimmt wird.

2. Verfahren nach Anspruch 1,
**dadurch gekennzeichnet,**
**dass** zur Bestimmung der Leistungsfähigkeit Herzfrequenz, Blutdruck, ergospirometrische Parameter oder Laktatkonzentration im Blut in Abhängigkeit von der Belastung des Menschen bestimmt wird.

3. Verfahren nach Anspruch 1 oder 2,
**dadurch gekennzeichnet,**
**dass** zur Bestimmung der Leistungsfähigkeit die individuelle anaerobe Schwelle des Menschen bestimmt wird.

4. Verfahren nach zumindest einem der vorhergehenden Ansprüche,
**dadurch gekennzeichnet,**
**dass** zur Bestimmung der Leistungsfähigkeit eine Normierung der oberhalb der individuellen anaeroben Schwelle gemessenen Leistung gemäß Laktat-Akkumulationsrate ΔA erfolgt.

5. Verfahren nach zumindest einem der vorhergehenden Ansprüche,
**dadurch gekennzeichnet,**
**dass** die individuelle anaerobe Schwelle nach Stegmann zur Bestimmung des individuellen Substratmetabolismus zu Grunde gelegt wird.

6. Verfahren nach zumindest einem der vorhergehenden Ansprüche,
**dadurch gekennzeichnet,**
**dass** bei einer Laktat-Akkumulationsrate ΔA gegen ΔA_{MAX} der Eiweißanteil der Ernährung bis mehrfach so hoch eingestellt wird als bei ΔA = 0 .

7. Verfahren nach zumindest einem der vorhergehenden Ansprüche zur nicht invasiven Bestimmung der Laktat-Akkumulationsrate ΔA, **gekennzeichnet durch** die Verfahrensschritte
- Messung der zeitabhängigen-Laktat-Konzentrationsänderung über die individuelle anaerobe Schwelle hinaus,
- Anpassen einer Messkurve an so gewonnene Messwerte, in der die Laktatkonzentration gegenüber der Zeit aufgetragen wird,
- Bestimmung einer ersten Steigung der Messkurve in einem der individuellen anaeroben Schwelle entsprechenden Zeitpunkt t_{IAT},
- Bestimmung zumindest einer weiteren Steigung aus der Messkurve zu einem Zeitpunkt tₓ mit tₓ > t_{IAT} und
- Subtraktion der zweiten Steigung von der ersten Steigung zur Bestimmung einer Differenz, die die Laktat-Akkumulationsrate ist.

8. Verfahren nach zumindest einem der vorhergehenden Ansprüche,
**dadurch gekennzeichnet,**
**dass** zur Bestimmung der Leistungsfähigkeit Belastungsarten wie Lauftests, Schwimmtests, Steptests, Ergometrieverfahren mit stufenweiser bzw. kontinuierlicher Belastungssteigerung mit und ohne Pausen genutzt werden.

9. Verfahren nach Anspruch 1,
**dadurch gekennzeichnet,**
der Kohlenhydrat-, Fett- und Eiweißanteil unter Zugrundlegung der für die Ernährung maßgeblichen belastungsspezifischen Verhältnisse von Kohlenhydrat, Fett- und Eiweißanteilen bestimmt wird.

## Claims

1. Method for adjusting the substrate metabolism of a person under consideration of one or more characteristic parameters such as blood pressure,
**characterized in**
**that** the performance capacity of the person being subjected to stress is not determined invasively by determining characteristic performance capacity parameters and that as a function of the determined performance capacity the carbohydrate and fat and protein percentage requirement is determined individually.

2. Method according to claim 1,
**characterized in**
**that**, for determining the performance capacity, heart rate, blood pressure, ergospirometric parameters or lactate concentration in the blood is determined as a function of the person's stress

3. Method according to claim 1 or 2,
**characterized in**
**that**, for determining the performance capacity, the person's individual anaerobic threshold is determined.

4. Method according to at least one of the preceding claims,
**characterized in**
**that**, for determining the performance capacity, a scaling of the performance measured above the individual anaerobic threshold occurs according to lactate accumulation rate ΔA.

5. Method according to at least one of the preceding claims,
**characterized in**
**that** the individual anaerobic threshold according to Stegmann is used as basis for the determination of the individual substrate metabolism.

6. Method according to at least one of the preceding claims,
**characterized in**
**that** with a lactate accumulation rate ΔA against ΔA_{MAX} the protein percentage of the nutrition is adjusted up to several times as high as with ΔA = 0.

7. Method according to at least one of the previous claims for the non-invasive determination of the lactate accumulation rate ΔA, **characterized by** the following procedural steps
- measuring the time-dependent lactate concentration change beyond the individual anaerobic threshold,
- adjusting a measurement curve to measurement values gained this way, in which the lactate concentration in relation to time is plotted,
- determining a first gradient of the measurement curve at a time t_{IAT} that corresponds to the individual anaerobic threshold,
- determining at least one additional gradient in the measurement curve at a time tₓ with tₓ > t_{IAT} and
- subtracting the second gradient from the first gradient to determine a difference, which represents the lactate accumulation rate ΔA.

8. Method according to at least one of the preceding claims,
**characterized in**
**that**, for determining the performance capacity, types of stress such as running tests, swimming tests, stepping tests, ergometry methods with gradual or continuous stress increase with and without breaks are used.

9. Method according to claim 1,
**characterized in**
**that** percentage of carbohydrate, fat and protein is determined on the basis of the stress-specific ratios of carbohydrate, fat and protein percentages that are decisive for the nutrition.

## Revendications

1. Procédé pour régler le métabolisme de substrat d'un être humain en prenant en considération un ou plusieurs paramètres caractéristiques tels que la tension artérielle,
**caractérisé en ce que**
la capacité fonctionnelle d'un être humain exposé à un effort est établie de manière non invasive par détermination de paramètres caractéristiques de la capacité fonctionnelle, et que les besoins en parts de glucides, lipides et protéines sont déterminés individuellement en fonction de la capacité fonctionnelle établie.

2. Procédé selon la revendication 1,
**caractérisé en ce que,**
pour établir la capacité fonctionnelle, la fréquence cardiaque, la tension artérielle, des paramètres ergospirométriques ou la concentration de lactate dans le sang sont analysés en fonction de l'effort imposé à l'être humain.

3. Procédé selon la revendication 1 ou 2,
**caractérisé en ce que**
le seuil anaérobique individuel de l'être humain est déterminé pour établir la capacité fonctionnelle.

4. Procédé selon au moins l'une des revendications précédentes,
**caractérisé en ce que,**
pour établir la capacité fonctionnelle, une normalisation de la performance mesurée au-dessus du seuil anaérobique individuel est effectuée selon le taux d'accumulation de lactate ΔA.

5. Procédé selon au moins l'une des revendications précédentes,
**caractérisé en ce que**
le seuil anaérobique individuel selon Stegmann est pris pour base pour déterminer le métabolisme de substrat.

6. Procédé selon au moins l'une des revendications précédentes,
**caractérisé en ce que,**
en présence d'un taux d'accumulation de lactate ΔA près de ΔA_{MAX}, le dosage de la part de protéines dans la nourriture est jusqu'à plusieurs fois plus élevé que quand ΔA = 0.

7. Procédé selon au moins l'une des revendications précédentes pour déterminer de manière non invasive le taux d'accumulation de lactate ΔA, **caractérisé par** les étapes suivantes
- mesurer la modification de la concentration de lactate en fonction du temps au-delà du seuil anaérobique individuel,
- ajuster une courbe de mesures aux valeurs mesurées ainsi obtenues, sur laquelle est portée la concentration de lactate en fonction du temps,
- déterminer une première pente de la courbe de mesures à un instant t_{IAT} correspondant au seuil anaérobique individuel,
- déterminer au moins une autre pente sur la courbe de mesures à un instant tₓ tel que tₓ > t_{IAT} et
- soustraire la deuxième pente de la première pente pour calculer une différence qui est le taux d'accumulation de lactate.

8. Procédé selon au moins l'une des revendications précédentes,
**caractérisé en ce que,**
pour établir la capacité fonctionnelle, des types d'efforts tels que tests de marche, tests de natation, tests d'escalier (step-tests), des méthodes ergométriques avec un accroissement de l'effort étagé ou continu avec et sans pauses peuvent être utilisés.

9. Procédé selon la revendication 1,
**caractérisé en ce que**
les parts de glucides, lipides et protéines dans la nourriture sont déterminées en se basant sur les proportions de glucides, lipides et protéines exigées par l'effort.
